# EUROPEAN PATENT SPECIFICATION

(11) **EP 3 492 071 B1**
(45) Date of publication and mention of the grant of the patent: **03.09.2025**
(21) Application number: 18206630.8
(22) Date of filing: 16.11.2018
(51) Int. Cl.: A61K 9/20

(54) **PHARMACEUTICAL COMPOSITION COMPRISING BENZOCAINE WITH ENHANCED STABILITY**
PHARMAZEUTISCHE ZUSAMMENSETZUNG MIT BENZOCAIN MIT ERHÖHTER STABILITÄT
COMPOSITION PHARMACEUTIQUE COMPRENANT DE LA BENZOCAÏNE PRÉSENTANT UNE STABILITÉ AMÉLIORÉE

(30) Priority: 29.11.2017 EP 17204447
(43) Date of publication of application: 05.06.2019
(73) Proprietor: Medice Arzneimittel Pütter GmbH & Co. KG, 58638 Iserlohn (DE)
(72) Inventor: Ammer, Richard, 58644 Iserlohn (DE); Buß, Uwe, 48167 Münster (DE)
(74) Representative: Isarpatent

(56) References cited:
- JP-A- 2010 083 826
- US-A- 3 832 460
- US-A1- 2008 014 290

## Description

The present invention refers to a pharmaceutical composition comprising at least benzocaine as pharmaceutically active agent.

### Background

Benzocaine is a local anesthetic commonly used as a topical pain reliever or in cough drops. It is the active ingredient in many over-the-counter anesthetic ointments such as products for oral ulcers. Pain is caused by the stimulation of free nerve endings. When the nerve endings are stimulated, sodium enters the neuron, causing depolarization of the nerve and subsequent initiation of an action potential. The action potential is propagated down the nerve toward the central nervous system, which interprets this as pain. Benzocaine acts to inhibit the voltage-dependent sodium channels (VDSCs) on the neuron membrane, stopping the propagation of the action potential.

Benzocaine is commonly formulated together with a variety of other compounds. Often the formulations will contain intentionally or unintentionally sugars, like glucose. For example, benzocaine is often formulated together with sorbitol which contains glucose as a contaminant. Glucose occurs as a contaminant in sorbitol since sorbitol is produced from glucose via a reductive reaction and the glucose is usually not entirely converted into sorbitol.

The inventors have found that benzocaine can react with sugars, like glucose, via a Maillard reaction during storage of formulations comprising benzocaine and a sugar resulting in the unwanted synthesis of benzocaine derivatives. The Maillard reaction is mainly known from the field of nutritional chemistry. The Maillard reaction is named for L. C. Maillard, who conducted pioneering work on sugar-amino acid condensations.1 The reaction scheme occurs in three phases (Saltmarch, M. et al. "Nonenzymatic Browning via the Maillard reaction in Foods", Diabetes, Vol. 31, Suppl. 3, June 1982). The first step in this scheme involves a condensation reaction between an amino group contributed by a free amino acid or a protein and the carbonyl group of a reducing sugar such as glucose, resulting in a product known as a Schiff's base as well as a molecule of water. Subsequent cyclization and isomerization under acidic conditions (Amadori rearrangement) results in a 1-amino-1-deoxy-2-ketose derivative. This component and its precursors are colorless. In the initial reaction both mono- and disaccharide reducing sugars can react. The reactivity of these reducing sugars varies; aldopentoses are more reactive than aldohexoses, which are, in turn, more reactive than disaccharides. The second, or intermediate reaction, phase involves the removal of amino groups from the reducing sugar complex with subsequent dehydration and cyclization, fragmentation, or amine condensation. Depending on environmental conditions such as temperature, three general pathways exist during this phase. In the first two pathways, hydroxymethyl furfural, reductones, dehydroreductones, and alphadicarbonyls result. These compounds can undergo Strecker degradation by a third pathway, at high temperatures. Complex polymerization reactions of the products from the second phase of the reaction scheme yield both soluble and insoluble brown melanoidin pigments in the third and final phase of the Maillard reaction.

The occurrence of the Maillard reaction in compositions comprising benzocaine is therefore not desirable. The Maillard reaction is accelerated in humid environments. Therefore, to stabilize benzocaine in compositions comprising sugars it would be required to 1) use highly expensive water vapor impermeable packaging material, 2) use very pure, and thus expensive excipients comprising a very low amount of sugars, or 3) to use a higher concentration of benzocaine when manufacturing the composition than was originally intended for benzocaine as the pharmaceutical agent in the composition.

US 3 832 460 A discloses a composition for the treatment of hypertrophied and hyperplastic oral tissue and the pain phenomena associated with this condition. In US 2008/014290 A1 a composition comprising an antacid, and a local, topical anesthetic used to relieve pain or discomfort associated with a sore throat is described. In JP 2010 083826 A a method for producing the solid preparation containing the oxicam-based compound is provided.

Accordingly, there is need for a composition that avoids the above disadvantages.

### Summary

It has been surprisingly found by the inventors that benzocaine can be stabilized in formulations comprising sugars by adding an amino acid as a stabilizing agent with at least one free amino group into the composition, wherein the stabilizing agent reacts with the reducing sugar in order to at least substantially reduce the Maillard reaction between the at least one nucleophilic amino group of the pharmaceutically active agent with the reducing sugar. Thus, the sugar preferentially reacts with the amino acid thereby consuming the reducing sugar and avoiding a reaction of the benzocaine with the reducing sugar. Therefore, the stability of the benzocaine in a composition is enhanced resulting in a longer shelf life of the composition. In addition, the use of expensive water vapor impermeable packaging, expensive excipients comprising low amounts of reducing sugars, and the use of increased amounts of benzocaine to compensate for the losses of benzocaine due to its reaction with reducing sugars can be avoided. Furthermore, the compositions of the invention allow incorporating higher amounts of reducing sugars thereby avoiding the use of sugar substitutes which may have unwanted side effects (e.g. laxative effects).

Accordingly, the invention relates to a pharmaceutical formulation, comprising:
benzocaine or a pharmaceutically acceptable salt thereof as a pharmaceutically active agent comprising at least one nucleophilic amino group,
a reducing sugar,
an excipient, and
an amino acid as a stabilizing agent with at least one free amino group, wherein the stabilizing agent reacts with the reducing sugar in order to at least substantially reduce the Maillard reaction between the at least one nucleophilic amino group of the pharmaceutically active agent with the reducing sugar, wherein the amino acid is selected from a group consisting of lysine, arginine, asparagine, glutamine or mixtures thereof, wherein the molar ratio of stabilizing agent to pharmaceutically active agent is between 0.5:1 and 6:1.

The reducing sugar can be a sugar with a free aldehyde radical, and preferably is a monosaccharide, a disaccharide or an oligosaccharide, preferably wherein the reducing sugar comprises glucose, fructose, lactose or mixtures thereof.

The stabilizing agent is contained in an amount which is sufficient to at least substantially reduce the Maillard reaction between the nucleophilic amino group of the pharmaceutically active agent and the reducing sugar, wherein the molar ratio of stabilizing agent to pharmaceutically active agent is between 0.5:1 and 6:1. It can be e.g. 1:1 to 5:1, e.g. 2:1 to 4:1.

The formulation can contain 0.1 - 5 wt.%, 0.5 - 4 wt.%, 1.0 - 3.0 wt.%, 1.5 - 2.5 wt.%, or 1.7 - 2.0 wt.% of the pharmaceutically active agent. The pharmaceutically active agent is contained in a pharmaceutically active amount to be selected depending on the particular use.

In the pharmaceutical formulation the pharmaceutical agent can be benzocaine, the reducing sugar can be glucose, and the stabilizing agent can be lysine, in particular L-lysine, further particularly L-lysine monohydrate.

The excipient can comprise sorbitol, talcum, sucrose stearate type III, saccharin-sodium x 2 H₂O, povidone K 25, carmellose-sodium.

The formulation can be optionally in the form of a tablet or lozenge, but also in any other form like liquids, ointments, etc.

The invention also relates to a method for preparing a pharmaceutical composition of the invention comprising the steps:
a. providing benzocaine or a pharmaceutically acceptable salt thereof as a pharmaceutically active agent comprising at least one nucleophilic amino group;
b. adding at least one excipient;
c. adding an amino acid as a stabilizing agent with at least one free amino group, wherein the amino acid is selected from a group consisting of lysine, arginine, asparagine, glutamine or mixtures thereof;
d. mixing the ingredients; and
e. obtaining the pharmaceutical composition,
wherein the stabilizing agent is contained in an amount which is sufficient to at least substantially reduce the Maillard reaction between the nucleophilic amino group of the pharmaceutically active agent and the reducing sugar, wherein the molar ratio of stabilizing agent to pharmaceutically active agent is between 0.5:1 and 6:1.

Furthermore, the invention relates to the use of an amino acid as a stabilizing agent in a pharmaceutical composition to at least substantially reduce the Maillard reaction between a nucleophilic amino group of benzocaine or a pharmaceutically acceptable salt thereof as a pharmaceutically active agent and a reducing sugar, wherein the stabilizing agent contains at least one free amino group, and wherein the stabilizing agent reacts with the reducing sugar in order to at least substantially reduce the Maillard reaction between the at least one nucleophilic amino group of the pharmaceutically active agent with the reducing sugar, wherein the amino acid is selected from a group consisting of lysine, arginine, asparagine, glutamine or mixtures thereof, wherein the stabilizing agent is contained in an amount which is sufficient to at least substantially reduce the Maillard reaction between the nucleophilic amino group of the pharmaceutically active agent and the reducing sugar, wherein the molar ratio of stabilizing agent to pharmaceutically active agent is between 0.5:1 and 6:1.

### Detailed Description

The formulation comprises benzocaine or a pharmaceutically acceptable salt thereof as a pharmaceutically active agent comprising at least one nucleophilic amino group, a reducing sugar, and an amino acid as a stabilizing agent. The amino acid comprises at least one free amino group, wherein the stabilizing agent reacts with the reducing sugar in order to at least substantially reduce the Maillard reaction between the at least one nucleophilic amino group of the pharmaceutically active agent with the reducing sugar. The amino acid is selected from a group consisting of lysine, arginine, asparagine, glutamine or mixtures thereof. The stabilizing agent is contained in an amount which is sufficient to at least substantially reduce the Maillard reaction between the nucleophilic amino group of the pharmaceutically active agent and the reducing sugar, wherein the molar ratio of stabilizing agent to pharmaceutically active agent is between 0.5:1 and 6:1. In one embodiment, further pharmaceutically active agents besides benzocaine may be contained.

Preferably, the molar ratio of stabilizing agent to pharmaceutically active agent is 1:1 and 5.5:1, 1:1 and 5:1, 1.5:1 and 4.5:1, 2:1 to 4:1, or 2.5.:1 and 3.5:1.

The formulation can contain 0.1 - 5 wt.% (weight/weight %), 0.5 - 4 wt.%, 1.0 - 3.0 wt.%, 1.5 - 2.5 wt.%, or 1.7 -2.0 wt.% of the pharmaceutically active agent. The formulation can contain 0.5 mg to 3 mg, 1.0 to 2.0, or 1.5 mg of the pharmaceutically active agent.

Benzocaine is ethyl 4-aminobenzoate and the ethyl ester of p-aminobenzoic acid (PABA):

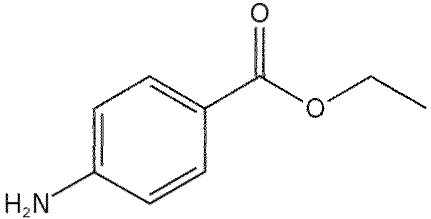

It contains one nucleophilic amino group.

The reducing sugar is a sugar (soluble carbohydrate) that is capable of acting as a reducing agent because it has a free aldehyde group or a free ketone group. Ketoses first tautomerize to aldoses before they can act as reducing sugars. Reducing sugars with a free aldehyde group can have a free aldehyde radical. The reducing sugar can be a monosaccharide, disaccharide, or an oligosaccharide or a mixture thereof. Preferably the reducing sugar is a monosaccharide. Preferred disaccharides can be lactose or maltose. Preferred monosaccharides can be glucose, fructose, or galactose. The formulation in a preferred embodiment comprises glucose and optionally a further reducing sugar like, for example, fructose, lactose or mixtures thereof. The reducing sugar of the invention can be a reducing sugar that is pharmaceutically acceptable and thus does occur naturally in nature.

The formulation comprises an amino acid as a stabilizing agent with at least one free amino group, wherein the stabilizing agent reacts with the reducing sugar in order to at least substantially reduce the Maillard reaction between the at least one nucleophilic amino group of the pharmaceutically active agent with the reducing sugar. The stabilizing agent is an L-amino acid selected from the group consisting of lysine, arginine, asparagine, glutamine, or mixtures thereof. Preferably, the stabilizing agent is (L-)lysine. Preferably, the stabilizing agent is a free amino acid, i.e. an amino acid that is not part of a peptide, protein, or not covalently bound to any other chemical compound at the time of the manufacture of the composition.

The formulation can further comprise an antibiotic or a mixture of antibiotics. In a preferred embodiment the formulation can be a polypeptide-antibiotic mixture or a cyclic polypeptide-antibiotic mixture from *Bacillus brevis.* In an even more preferred embodiment the cyclic polypeptide-antibiotic mixture is tyrothricin.

The mass ratio of the pharmaceutical active agent to the antibiotic can be between 0.5:1 and 6:1, 1:1 and 5.5:1, 1:1 and 5:1, 1.5:1 and 4.5:1, 2:1 to 4:1, or 2.5.:1 and 3.5:1.

The formulation can comprise 0,1 wt.% to 1 wt.%, 0.2 wt.% to 0.8 wt.%, 0.3 wt.% to 0.7 wt.%, 0.4 to 0.6 wt.% of an antibiotic or a mixture of antibiotics.

The formulation can further comprise a pharmaceutically acceptable disinfectant having antimicrobial activity, for example, a cationic quaternary ammonium compound like cetylpyridiniumchlorid or benzalkonium chloride. A preferred disinfectant is benzalkonium chloride.

The mass ratio of the pharmaceutical active agent to the disinfectant can be between 0.5:1 and 0.5:1 and 3:1, 0.75:1 to 2.5:1, or 1:1 and 2:1.

The formulation can further comprise an excipient, preferably a pharmaceutical acceptable excipient. The choice of excipient will to a large extent depend on factors such as the particular mode of administration, the effect of the excipient on solubility and stability, and the nature of the dosage form.

The excipient can be a sugar substitute, preferably a sugar alcohol, a saccharin (e.g. saccharin-sodium x 2 H₂O), aspartame, cyclamate, thaumatin, or acesulfam, or a combination thereof. Preferably, the formulation comprises sorbitol and optionally a further sugar substitute or only sorbitol as a sugar substitute.

The sugar substitute can be present in amount of 50 to 99 wt.%, 60 to 95 wt.%,, 80-93 wt.% (, or 85-90 wt.% in the formulation.

Further pharmaceutically acceptable excipients include, but are not limited to, talcum, sucrose stearate (preferably type III), povidone (preferably povidone K 25), carmellose sodium, ion exchangers, alumina, aluminum stearate, lecithin, self-emulsifying drug delivery systems (SEDDS) such as d-a-tocopherol polyethylene glycol 1000 succinate, surfactants used in pharmaceutical dosage forms such as Tweens or other similar polymeric delivery matrices, serum proteins, such as human serum albumin, buffer substances such as phosphates, , sorbic acid, potassium sorbate, partial glyceride mixtures of saturated vegetable fatty acids, water, salts or electrolytes, such as protamine sulfate, disodium hydrogen phosphate, potassium hydrogen phosphate, sodium-chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, and wool fat. Cyclodextrins such as α-, β, and γ-cyclodextrin, or chemically modified derivatives such as hydroxyalkylcyclodextrins, including 2- and 3- hydroxypropyl-b-cyclodextrins, or other solubilized derivatives can also be used.

Further pharmaceutically acceptable excipients include flavorings, e.g. essential oils. Preferred essential oils are peppermint oil, menthol, lavender oil, tear tree oil, patchouli oil, citrus and eucalyptus oil or a combination thereof. An even more preferred essential oil is peppermint oil.

The formulation can comprise 0.1 to 5.0 wt%, 0.2 to 4.0 wt.%, 0.3 to 3.0 wt.%, 0.4 to 2.0% flavorings.

Preferably, the excipient comprises sorbitol, talcum, sucrose stearate type III, saccharin-sodium x 2 H₂O, povidone K 25, and carmellose-sodium.

The formulation can comprise 0.5 wt.% to 1.5 wt.% or 0.9 to 1.1 wt.% sorbitol.

The formulation can comprise 70.0 wt.% to 90 wt.% or 75.0 wt.% to 85 wt.% talcum.

The formulation can comprise 15.0 wt.% to 25.0 wt.% or 18.0 wt.% to 22.0 wt.% sucrose stearate type III.

The formulation can comprise 0.5 wt.% to 1.5 wt.% or 0.9 wt.% to 1.1 wt.% saccharin-sodium x 2 H₂O.

The formulation can comprise 7.5 wt.% to 12.5 wt.% or 9.0 wt.% to 11.0 wt.% povidone K25.

The formulation can comprise 7.5 wt.% to 12.5 wt.% or 9.0 wt.% to 11.0 wt.% carmellose-sodium.

A formulation can be, in one embodiment, formulated into suitable pharmaceutical preparations such as solutions, suspensions, tablets, lozenges, dispersible tablets, pills, capsules, powders, aerosols, sustained release formulations or elixirs, for oral administration or in sterile solutions, as well as transdermal ointments, creams, gels, pastes, and patch preparations and dry powder inhalers (see, e.g., Ansel Introduction to Pharmaceutical Dosage Forms, Fourth Edition 1985, 126). Preferred, is that the formulation is a lozenge or tablet.

The formulation, for example, in the form of a tablet or lozenge, can have a weight of 800 to 1200 mg, 900 to 1100 mg, or 950 to 1050 mg, or 1000 mg.

The formulation of the invention can be used in various medical indications and methods of treatment.

In particular, the formulation can be used for local anesthesia of oral and pharyngeal mucous membranes (for example, sore throat, cold sores, mouth ulcers, toothache, sore gums, denture irritation, etc.).

The formulation of the invention can be used for relieving ear conditions (for example, otitis media, swimmers ear, etc.).

The formulation of the invention can be used in dietary products useful for weight reduction.

The formulation of the invention can be used in the prevention of premature ejaculation. In particular, the composition can be used as a cream or ointment or as a coating for the inside of a condom.

The formulation of the invention can be used for reducing orthodontic pain. For example, the formulation can be comprised in mucoadhesive patches.

The formulation of the invention can be used for the treatment of skin after insect bites (e.g. by mosquitos).

The formulation can be prepared by any conventional method for producing pharmaceutical formulations.

Thus, a method of preparing a pharmaceutical composition according to any of the preceding claims can comprise the steps of providing benzocaine or a pharmaceutically acceptable salt thereof as a pharmaceutically active agent comprising at least one nucleophilic amino group; adding at least one of the excipients as described above; adding an amino acid as described above as a stabilizing agent with at least one free amino group; mixing the ingredients; and obtaining the pharmaceutical formulation of the invention. The sequence of steps may be varied. For example, the excipients may be provided in a first step and the benzocaine added only in a subsequent step, and optionally further excipients may be added to the composition in even more subsequent steps.

### Examples

### Example 1: Production of formulation

The following ingredients were used in the production of four different formulations of a tablet:

| Ingredient | [g] ingredient per 1000 g |
|---|---|
| Carmellose-sodium | 10.0 |
| Peppermint oil | 5.0 |
| Povidon K 25 | 10.0 |
| Sucrose stearate type III | 20.0 |
| Saccharin-sodium x ₂ H2O | 1.0 |
| Talkum | 79.9 |
| L-Lysinmonhydrate | 0/1.5/3.0/10.0 |
| Sorbitol | 872.7/870.7/869,2.7/859.2 |
| Benzocain | 1.9 |
| Sum | 1000 q |

The ingredients were used to provide tablets of 1000 mg each using the following method:
Saccharin sodium, benzocaine, and povidone K25 are solved in 96% ethanol at room temperature to provide drug solution 1.

Drug solution 1 is sprayed onto about 50% of the sorbitol in a fluid bed system and dried at 25-35°C until the ethanol is evaporated to provide dried material 1.

The dried material 1 is mixed in a rotary-drum with the remaining about 50% of the sorbitol. Then, the peppermint oil is added. The rotary speed is 5-10 rpm and the mixing time about 1 hour at room temperature to provide dried material 2.

Carmellose sodium, sucrose stearate Type III, talc, and L-Lysinmonhydrate are added to dried material in the rotary-drum. The rotary speed is 5-10 rpm and the mixing time about 0.1-0.5 hours at room temperature to provide dried material 2 to obtain a granulated material.

The granulated material is sieved and subsequently tableted using a rotary tablet machine to provide tablets of about 1000 mg.

The tablets of example are stored 25°C at 60% relative humidity for 33 months before analysis.

### Example 2: Analysis method

The analysis is performed using high pressure liquid chromatography (HPLC) according to Ph. Eur. 2.2.29.

The separation column was a Kinetex C18, 150 mm x 4.6 mm. Eluent A was 20 mM sodium acetate in water, pH adjusted to 4.7 with acetic acid. Eluent B was acetontril. The applied gradient starts at 14 vol.-% of eluent B and ends at 80 vol.% eluent B at rate of about 1ml/min. Sample size was 15 µl.

The analysis was performed at 8°C via UV detection at 288 nm and a reference 400 nm.

0.5 g of the tablets of example 1 are thoroughly dissolved in 50 ml 25 % acetonitrile. The reference was 1.5 mg benzocaine in 100 ml of 25 % acetonitrile.

The reference showed at peak at about 19 ml of the elution volume which was determined to be undegraded benzocaine. Samples stored for 1 month showed additional peaks between 6.5 ml and 13 ml of the elution volume (B1-B12) with a pronounced peak at 7.5 ml which was designated as peak B4 and considered to represent a Maillard product of benzocaine and glucose. Peaks B1-B12 were considered as degradation products of benzocaine.

### Example 3: Results

| Concentration of L-Lysinmonohydrate [mg] | Sum of impurities [wt/wt-%] | B4 [wt/wt-%] | Concentration of Benzocain [wt/wt-%] |
|---|---|---|---|
| 0 | 4,19 | 2,31 | 50,2 |
| 1,5 | 1,35 | 0,74 | 50,6 |
| 3,0 | 0,97 | 0,59 | 48,7 |
| 10,0 | 0,66 | 0,44 | 50,2 |

It was found that the presence of L-Lysinmonohydrate in a quantitative manner prevents the formation of impurities B1-B12 and, in particular, the formation of impurity B4 which is considered to represent the Maillard product of benzocaine and glucose.

## Claims

1. A pharmaceutical formulation comprising:
benzocaine or a pharmaceutically acceptable salt thereof as a pharmaceutically active agent comprising at least one nucleophilic amino group,
a reducing sugar,
an excipient, and
an amino acid as a stabilizing agent with at least one free amino group, wherein the stabilizing agent reacts with the reducing sugar in order to at least substantially reduce the Maillard reaction between the at least one nucleophilic amino group of the pharmaceutically active agent with the reducing sugar, wherein the amino acid is selected from a group consisting of lysine, arginine, asparagine, glutamine or mixtures thereof, wherein the stabilizing agent is contained in an amount which is sufficient to at least substantially reduce the Maillard reaction between the nucleophilic amino group of the pharmaceutically active agent and the reducing sugar, wherein the molar ratio of stabilizing agent to pharmaceutically active agent is between 0.5:1 and 6:1.

2. The pharmaceutical formulation according to claim 1, wherein the amino acid is lysine.

3. The pharmaceutical formulation according to any of the preceding claims, wherein the reducing sugar is a sugar with a free aldehyde radical, and preferably is a monosaccharide, a disaccharide or an oligosaccharide, preferably wherein the reducing sugar comprises glucose, fructose, lactose or mixtures thereof.

4. The pharmaceutical formulation according to any of the preceding claims, wherein the molar ratio of stabilizing agent to pharmaceutically active agent is. 1:1 to 5:1, e.g. 2:1 to 4:1.

5. The pharmaceutical formulation according to any of the preceding claims, wherein the formulation contains 0.1 - 5 wt.%, 0.5 - 4 wt.%, 1.0 - 3.0 wt.%, 1.5 - 2.5 wt.%, or 1.7 - 2.0 wt.% of the pharmaceutically active agent.

6. The pharmaceutical formulation according to any of the preceding claims, wherein the pharmaceutical agent is benzocaine, the reducing sugar is glucose, and the stabilizing agent is lysine.

7. The pharmaceutical formulation according to any of the preceding claims, wherein the excipient comprises sorbitol, talcum, sucrose stearate type III, saccharin-sodium x 2 H₂O, povidone K 25, carmellose-sodium.

8. The pharmaceutical formulation according to any of the preceding claims, wherein the formulation is in the form of a tablet.

9. A method of preparing a pharmaceutical formulation according to any of the preceding claims comprising the steps of
a. providing benzocaine or a pharmaceutically acceptable salt thereof as a pharmaceutically active agent comprising at least one nucleophilic amino group;
b. adding at least one excipient;
c. adding an amino acid as a stabilizing agent with at least one free amino group, wherein the amino acid is selected from a group consisting of lysine, arginine, asparagine, glutamine or mixtures thereof;
d. mixing the ingredients; and
e. obtaining the pharmaceutical composition,
wherein the stabilizing agent is contained in an amount which is sufficient to at least substantially reduce the Maillard reaction between the nucleophilic amino group of the pharmaceutically active agent and the reducing sugar, wherein the molar ratio of stabilizing agent to pharmaceutically active agent is between 0.5:1 and 6:1.

10. Use of an amino acid as a stabilizing agent in a pharmaceutical composition to at least substantially reduce the Maillard reaction between a nucleophilic amino group of benzocaine or a pharmaceutically acceptable salt thereof as a pharmaceutically active agent and a reducing sugar, wherein the stabilizing agent contains at least one free amino group, and wherein the stabilizing agent reacts with the reducing sugar in order to at least substantially reduce the Maillard reaction between the at least one nucleophilic amino group of the pharmaceutically active agent with the reducing sugar, wherein the amino acid is selected from a group consisting of lysine, arginine, asparagine, glutamine or mixtures thereof, wherein the stabilizing agent is contained in an amount which is sufficient to at least substantially reduce the Maillard reaction between the nucleophilic amino group of the pharmaceutically active agent and the reducing sugar, wherein the molar ratio of stabilizing agent to pharmaceutically active agent is between 0.5:1 and 6:1.

11. Use according to claim 10 wherein the stabilizing agent is lysine, and wherein the reducing sugar is glucose.

## Patentansprüche

1. Pharmazeutische Formulierung, umfassend:
Benzocain oder ein pharmazeutisch verträgliches Salz davon als pharmazeutisch wirksames Mittel, das mindestens eine nukleophile Aminogruppe enthält,
einen reduzierenden Zucker,
einen Hilfsstoff und
eine Aminosäure als Stabilisierungsmittel mit zumindest einer freien Aminogruppe,
wobei das Stabilisierungsmittel mit dem reduzierenden Zucker reagiert, um die Maillard-Reaktion zwischen der zumindest einen nukleophilen Aminogruppe des pharmazeutisch wirksamen Mittels mit dem reduzierenden Zucker zumindest wesentlich zu reduzieren, wobei die Aminosäure ausgewählt ist aus der Gruppe, die aus Lysin, Arginin, Asparagin, Glutamin oder Mischungen davon besteht, wobei das Stabilisierungsmittel in einer Menge enthalten ist, die ausreicht, um die Maillard-Reaktion zwischen der nukleophilen Aminogruppe des pharmazeutisch wirksamen Mittels und dem reduzierenden Zucker zumindest wesentlich zu reduzieren, wobei das molare Verhältnis von Stabilisierungsmittel zu pharmazeutisch wirksamem Mittel zwischen 0,5:1 und 6:1 liegt.

2. Pharmazeutische Formulierung nach Anspruch 1, wobei die Aminosäure Lysin ist.

3. Pharmazeutische Formulierung nach einem der vorhergehenden Ansprüche, wobei der reduzierende Zucker ein Zucker mit einem freien Aldehydradikal ist und vorzugsweise ein Monosaccharid, ein Disaccharid oder ein Oligosaccharid ist, wobei der reduzierende Zucker vorzugsweise Glucose, Fructose, Lactose oder Mischungen davon umfasst.

4. Pharmazeutische Formulierung nach einem der vorhergehenden Ansprüche, wobei das molare Verhältnis von Stabilisator zu pharmazeutisch wirksamem Mittel 1:1 bis 5:1, z.B. 2:1 bis 4:1 beträgt.

5. Pharmazeutische Formulierung nach einem der vorhergehenden Ansprüche, wobei die Formulierung 0,1 - 5 Gew.-%, 0,5 - 4 Gew.-%, 1,0 - 3,0 Gew.-%, 1,5 - 2,5 Gew.-% oder 1,7 - 2,0 Gew.-% des pharmazeutisch wirksamen Mittels enthält.

6. Pharmazeutische Formulierung nach einem der vorhergehenden Ansprüche, wobei der pharmazeutische Wirkstoff Benzocain ist, der reduzierende Zucker Glucose ist und das Stabilisierungsmittel Lysin ist.

7. Pharmazeutische Formulierung nach einem der vorhergehenden Ansprüche, wobei der Hilfsstoff Sorbitol, Talkum, Saccharosestearat Typ III, Saccharin-Natrium x 2 H₂O, Povidon K 25, Carmellose-Natrium umfaßt.

8. Pharmazeutische Formulierung nach einem der vorhergehenden Ansprüche, wobei die Formulierung in Form einer Tablette vorliegt.

9. Verfahren zur Herstellung einer pharmazeutischen Formulierung nach einem der vorangehenden Ansprüche, umfassend die Schritte
a. Bereitstellen von Benzocain oder eines pharmazeutisch annehmbaren Salzes davon als pharmazeutisch wirksames Mittel, das mindestens eine nukleophile Aminogruppe enthält;
b. Zugabe mindestens eines Hilfsstoffes;
c. Zugabe einer Aminosäure als Stabilisierungsmittel mit mindestens einer freien Aminogruppe, wobei die Aminosäure aus einer Gruppe ausgewählt ist, die aus Lysin, Arginin, Asparagin, Glutamin oder Mischungen davon besteht;
d. Mischen der Bestandteile; und
e. Erhalten der pharmazeutischen Zusammensetzung,
wobei das Stabilisierungsmittel in einer Menge enthalten ist, die ausreicht, um die Maillard-Reaktion zwischen der nukleophilen Aminogruppe des pharmazeutisch wirksamen Mittels und dem reduzierenden Zucker zumindest wesentlich zu reduzieren, wobei das molare Verhältnis von Stabilisierungsmittel zu pharmazeutisch wirksamem Mittel zwischen 0,5:1 und 6:1 liegt.

10. Verwendung einer Aminosäure als Stabilisierungsmittel in einer pharmazeutischen Zusammensetzung, um die Maillard-Reaktion zwischen einer nukleophilen Aminogruppe von Benzocain oder einem pharmazeutisch akzeptablen Salz davon als pharmazeutisch wirksamem Mittel und einem reduzierenden Zucker zumindest wesentlich zu reduzieren, wobei das Stabilisierungsmittel mindestens eine freie Aminogruppe enthält und wobei das Stabilisierungsmittel mit dem reduzierenden Zucker reagiert, um die Maillard-Reaktion zwischen der mindestens einen nukleophilen Aminogruppe des pharmazeutisch wirksamen Mittels mit dem reduzierenden Zucker zumindest wesentlich zu reduzieren, wobei die Aminosäure aus einer Gruppe ausgewählt ist, die aus Lysin, Arginin, Asparagin, Glutamin oder Mischungen davon besteht, wobei das Stabilisierungsmittel in einer Menge enthalten ist, die ausreicht, um die Maillard-Reaktion zwischen der nukleophilen Aminogruppe des pharmazeutisch wirksamen Mittels und dem reduzierenden Zucker zumindest wesentlich zu reduzieren, wobei das molare Verhältnis von Stabilisierungsmittel zu pharmazeutisch aktivem Mittel zwischen 0,5:1 und 6:1 liegt.

11. Verwendung nach Anspruch 10, wobei das Stabilisierungsmittel Lysin ist und der reduzierende Zucker Glucose ist.

## Revendications

1. Formulation pharmaceutique comprenant :
de la benzocaïne ou un de ses sels pharmaceutiquement acceptables en tant qu'agent pharmaceutiquement actif comprenant au moins un groupe amino nucléophile,
un sucre réducteur,
un excipient, et
un acide aminé comme agent stabilisateur avec au moins un groupe aminé libre, dans lequel l'agent stabilisateur réagit avec le sucre réducteur afin de réduire au moins substantiellement la réaction de Maillard entre au moins un groupe aminé nucléophile de l'agent pharmaceutique actif avec le sucre réducteur, dans lequel l'acide aminé est choisi dans un groupe consistant en lysine, l'arginine, l'asparagine, la glutamine ou leurs mélanges, dans lequel l'agent stabilisant est contenu en quantité suffisante pour réduire au moins substantiellement la réaction de Maillard entre le groupe amino nucléophile de l'agent pharmaceutiquement actif et le sucre réducteur,
dans lequel le rapport molaire entre l'agent stabilisant et l'agent pharmaceutiquement actif est compris entre 0.5:1 et 6:1.

2. Formulation pharmaceutique selon la revendication 1, dans laquelle l'acide aminé est la lysine.

3. Formulation pharmaceutique selon l'une quelconque des revendications précédentes, dans laquelle le sucre réducteur est un sucre avec un radical aldéhyde libre, et de préférence est un monosaccharide, un disaccharide ou un oligosaccharide, de préférence dans laquelle le sucre réducteur comprend du glucose, du fructose, du lactose ou des mélanges de ceux-ci.

4. Formulation pharmaceutique selon l'une des revendications précédentes, dans laquelle le rapport molaire entre l'agent stabilisant et l'agent pharmaceutiquement actif est. 1:1 à 5:1, par exemple 2:1 à 4:1.

5. La formulation pharmaceutique selon l'une des revendications précédentes, dans laquelle la formulation contient 0,1 - 5 % en poids, 0,5 - 4 % en poids, 1,0 - 3,0 % en poids, 1,5 - 2,5 % en poids, ou 1,7 - 2,0 % en poids de l'agent pharmaceutiquement actif.

6. La formulation pharmaceutique selon l'une des revendications précédentes, dans laquelle l'agent pharmaceutique est la benzocaïne, le sucre réducteur est le glucose et l'agent stabilisant est la lysine.

7. Formulation pharmaceutique selon l'une quelconque des revendications précédentes, dans laquelle l'excipient comprend du sorbitol, du talc, du stéarate de saccharose de type III, de la saccharine sodique x 2 H₂O, de la povidone K 25, de la carmellose sodique.

8. Formulation pharmaceutique selon l'une des revendications précédentes, dans laquelle la formulation se présente sous la forme d'un comprimé.

9. Méthode de préparation d'une formulation pharmaceutique selon l'une des revendications précédentes, comprenant les étapes suivantes
a. fournir de la benzocaïne ou un de ses sels pharmaceutiquement acceptables en tant qu'agent pharmaceutiquement actif comprenant au moins un groupe amino nucléophile ;
b. ajouter au moins un excipient ;
c. ajouter un acide aminé comme agent stabilisateur avec au moins un groupe aminé libre, l'acide aminé étant choisi dans le groupe constitué par la lysine, l'arginine, l'asparagine, la glutamine ou leurs mélanges ;
d. mélanger les ingrédients ; et
e. obtenir la composition pharmaceutique,
dans laquelle l'agent stabilisant est contenu en quantité suffisante pour réduire au moins substantiellement la réaction de Maillard entre le groupe amino nucléophile de l'agent pharmaceutiquement actif et le sucre réducteur, le rapport molaire entre l'agent stabilisant et l'agent pharmaceutiquement actif étant compris entre 0,5:1 et 6:1.

10. Utilisation d'un acide aminé comme agent stabilisant dans une composition pharmaceutique pour réduire au moins substantiellement la réaction de Maillard entre un groupe aminé nucléophile de la benzocaïne ou d'un de ses sels pharmaceutiquement acceptables en tant qu'agent pharmaceutiquement actif et un sucre réducteur, dans laquelle l'agent stabilisant contient au moins un groupe aminé libre, et dans laquelle l'agent stabilisant réagit avec le sucre réducteur afin de réduire au moins substantiellement la réaction de Maillard entre le groupe aminé nucléophile au moins de l'agent pharmaceutiquement actif et le sucre réducteur, dans lequel l'acide aminé est choisi dans le groupe constitué par la lysine, l'arginine, l'asparagine, la glutamine ou leurs mélanges, dans lequel l'agent stabilisant est contenu en quantité suffisante pour réduire au moins substantiellement la réaction de Maillard entre le groupe aminé nucléophile de l'agent pharmaceutiquement actif et le sucre réducteur, dans lequel le rapport molaire entre l'agent stabilisant et l'agent pharmaceutiquement actif est compris entre 0.5:1 et 6:1.

11. Utilisation selon la revendication 10 dans laquelle l'agent stabilisant est la lysine, et dans laquelle le sucre réducteur est le glucose.
